Europäisches Patentamt

European Patent Office

Office européen des brevets

(19)

(11) Publication number : **0 628 317 A1**

(12) # EUROPEAN PATENT APPLICATION

(21) Application number : **94304125.1**

(22) Date of filing : **08.06.94**

(51) Int. Cl.⁵ : **A61L 2/26**

(30) Priority : **09.06.93 US 73424**

(43) Date of publication of application :
**14.12.94 Bulletin 94/50**

(84) Designated Contracting States :
**DE FR GB**

(71) Applicant : **PyMaH Corporation**
**500 Route 202 North**
**Flemington, New Jersey 08822 (US)**

(72) Inventor : **Ignacio, Ramon T.**
**2306 Rebecca Street**
**West Covina, California 91792 (US)**
Inventor : **Thackston, Thomas**
**404 East Oak Avenue**
**Morrestown, New Jersey 08057 (US)**

(74) Representative : **Belcher, Simon James**
**Urquhart-Dykes & Lord**
**Tower House**
**Merrion Way**
**Leeds LS2 8PA (GB)**

(54) **Simplified sterilizer vacuum test pack.**

(57)   A sterilization autoclave vacuum testing device which comprises a transparent gas impervious layer 1 adhered to an indicator layer. The indicator layer comprising a steam porous medium having imprinted there on a pattern utilizing a thermochromic ink composition. The indicator layer 2 is adhesively bonded to a porous barrier layer substrate 3 having a predetermined porosity, sufficient to prevent a complete and uniform colour change of the thermochromic ink at a vacuum of at least about 711 mm of mercury (95 kPa) at a predetermined temperature.

EP 0 628 317 A1

This invention relates to a device for testing the effectiveness of a vacuum in a steam sterilization autoclave. In particular it relates to a simplified device which does not require extensive hand labour to construct it.

It is well known that heat will destroy microorganisms. The presence of moisture accelerates this destruction by denaturing or coagulation of the protein making up the microorganism. The importance of moist heat in the sterilization process has been known for about 100 years. While Pasteur had established the fact that temperatures above the boiling point of water were required to kill many organisms, it was not until 1880 that a steam pressure sterilizer was developed. However, there was no significant notice of the need for sterilization of hospital equipment and fabrics until the introduction of first antiseptic and then aseptic surgery.

About the turn of the century manually controlled sterilizing autoclaves were introduce into hospitals. Their efficiency left much to be desired, but nonetheless, this was a definite step forward. As a result of advances in the food industry a fuller understanding of parameters required for spore kill was developed, and it was determined that the order of death was logarithmic.

The most significant aspect in the development of safe and effect sterilization techniques was the recognition of the presence of moisture in the killing of microorganisms. Most microorganisms contain sufficient moisture so that moderate heat alone, eg 80°-100°C, will destroy the microorganism. Most bacterial spores, however, contain substantially no moisture. Their destruction by dry heat alone requires elevated temperatures in excess of 150°C. Such excessive temperatures can result in the destruction of the article to be sterilized, or otherwise seriously limit its useful life. Hence, in the hospital arena pathogenic spores are destroyed using a steam atmosphere in autoclaves.

While the first effective steam sterilizers were conventionally operated at about 250°F (120°C) for sterilization cycles of about 12 to 15 minutes, the preferred sterilization conditions are about 270°F (130°C) for about 3 minutes.

Since steam is essential to the effective operation of the sterilization process it is important to ensure that steam comes into contact with the materials to be sterilized. It is common practice in hospitals to overwrap goods to be sterilized with a cloth wrapping to ensure that the sterile material will not become contaminated after removal from the autoclave and before use.

Because this overwrap restricts access of steam to the goods to be sterilized it is essential to ensure that the overwrap does not prevent the goods from coming into contact with the steam during the sterilization cycle. This is accomplished by drawing a vacuum on the steam sterilization autoclave to remove air, the consequence of which is that the void created by displaced air is filled by the steam introduced into the autoclave. Those skilled in the art recognize that an effective vacuum is critical to the effective operation of the sterilizer. To that end various devices for measuring the effectiveness of the vacuum cycle of the sterilization process have been developed.

The classical industry standard for determining whether or not the autoclave is functioning properly is a test known as the Bowie-Dick test. The test utilizes a test pack comprising an indicator which is steam sensitive contained in the same type of wrapping utilized in preparing goods for sterilization. The test pack comprises a steam sensitive indicator sheet having a design imprinted thereon in an ink which will change colour in the presence of steam at the temperatures utilized in steam sterilization processes.

Because each test pack must be independently prepared it is an inconvenient test. Furthermore, the hand wrapped test packs can vary in porosity because of differences in tightness of the wrap. These and other variables make the test of questionable reproducibility.

In order to avoid the problems created in utilizing the Bowie-Dick test to measure the vacuum efficiency of steam sterilization autoclaves, various test packs utilizing paper pads have been developed. US-4579715 discloses a device comprising a porous bundle having a sheet imprinted with a steam sensitive ink interposed between the porous sheets of the pack. Steam impervious sheets are place on the top and bottom of the pack thereby leaving only the edges of the pack porous to the entry of steam. The porous bundle is overwrapped in a porous paper wrapping.

US-4756795 discloses a similar test pack having the improvement comprising a foam layer being positioned between the impervious top layer and the porous layers. See also US-4692307.

While the devices of US-4579715, US-4756795 and US-4692307 give more reproducible results than those which can be obtained by a hand wrapped Bowie-Dick test pack they suffer from the disadvantage that the packs require hand lay up of the various layers with a consequent increase in cost. Furthermore, they require hand overwrapping with the outer porous layer which serves to hold the pack together, and additionally are somewhat bulky though less so than the prior art cloth wrapped Bowie-Dick test packs.

What is required is a test pack which can be put together without the extensive hand labour required for prior art test packs while at the same time giving the same assurance of test reliability of prior art devices.

It has now been found that a test pack which effectively measures the vacuum efficiency of a steam sterilization autoclave can be prepared by laminating a pack of porous material having a predetermined porosity to an indicator sheet having imprinted thereon an ink which is steam sensitive. A gas impervious layer is bonded to the indicator sheet to prevent direct contact of the indicator sheet with the steam. The vacuum tester of this invention requires no overwrap, and can be read directly when it is removed from the autoclave.

Accordingly, this invention provides an improved Bowie-Dick test device which comprises a multiplicity of porous sheets bonded to an indicator layer. The indicator layer is protected from direct contact with steam by a gas impervious layer bonded thereto. The test pack comprises a single composite structure which requires no overwrapping and can be read directly when removed from the autoclave.

The present invention will now be described, by way of example only, with reference to the accompanying drawings, in which:

Figure 1 shows the structure of the Improved Vacuum Test Pack of this invention.

Figure 2 shows an illustrative prior art configuration of the ink pattern on the indicator layer of the device of the invention.

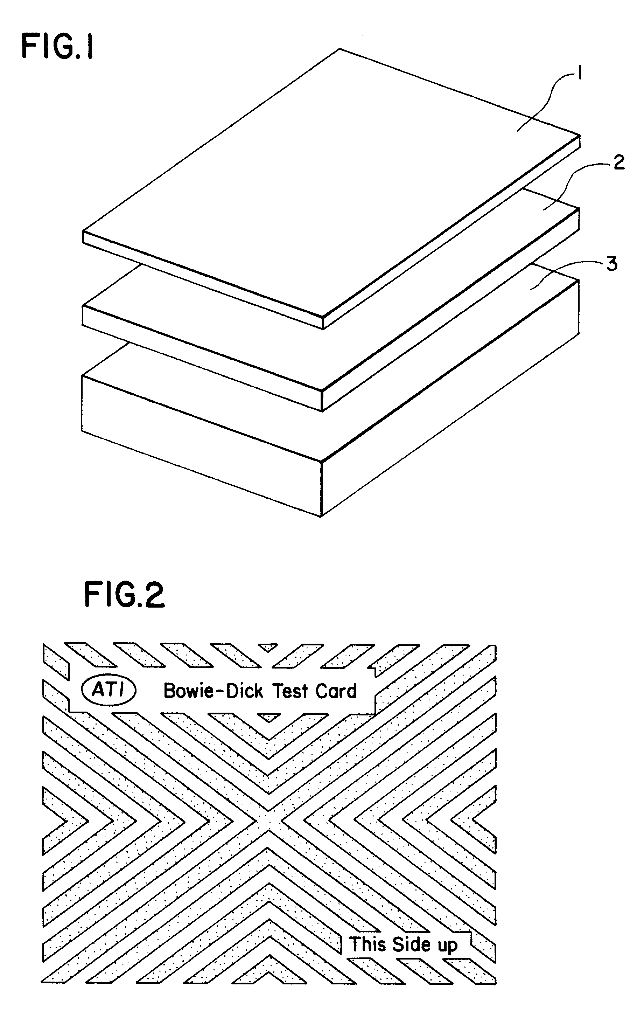

Referring to Figure 1, an impervious layer of transparent polymeric film, 1, is bonded to an indicator layer, 2, which in turn is bonded to at least one porous barrier layer, 3. The barrier layers can be adhesively bonded to one another and to the indicator layer. The impervious layer 1, can be either heat sealed or adhesively bonded to the indicator layer.

The impervious layer 1, can be made of any transparent material which will withstand the sterilization conditions which comprise steam at a temperature of about 120°C or about 134°C. Of course, those skilled in the art having access to this disclosure will appreciate that the impervious layer 1, should be capable of withstanding temperatures significantly higher than the sterilization temperature since temperature excursions may occur in the sterilization cycle. Preferably, the impervious layer will have a glass transition temperature of at least 200°C, eg 280°C.

The gas impervious layer 1, can be mica, glass, or a natural or synthetic transparent polymeric material. Illustrative, non-limiting examples of polymeric materials useful in the practice of this invention are polyesters, polycarbonates, polyphenylene oxides and polystyrene. A typical polyester useful in the practice of this invention is a 0.5 mil (0.01 mm) film of Supercold Seal™ polyester supplied by the Fasson Film Division of Avery/Denison Company. This film is supplied with a coating of pressure sensitive acrylic adhesive.

While the gas impervious polymeric material can be heat sealed to the indicator layer, 2, it is preferred that it be adhesively bonded. The adhesive can overcome slight irregularities in the surface of the indicator layer, 2, or the porous barrier layer.

Although the thickness of the gas impervious layer, 1, is not critical, it is preferably not be so thick that the adhesive layer, 4, cannot correct for surface irregularities because of the inflexibility of the gas impervious layer, 1. A desirable range of thicknesses for the gas impervious polymeric material layer, 1, is about 0.5 to about 1.25 mils (0.01 to 0.03 mm).

The indicator layer can be any porous material which will permit the steam to penetrate freely. Typically it will be a thin sheet of paper, although it may comprise a paper board material of several mils (hundredths of mm) in thickness.

In one embodiment, the indicator layer can be a polymeric film with high water vapour transmission properties, e.g., nylon or ethylene vinylacetate.

In another embodiment the indicator layer, per se, can be eliminated and a thermochromic ink pattern can be imprinted on the underside of the gas impervious layer, or onto the top of the barrier layer. Where the barrier layer comprises a multiplicity of layers adhered together, one of the layers making up the barrier layer, 3, can double as the indicator layer, 2.

The porous barrier layer, 3, serves to restrict the flow of steam to the indicator layer, 2. While a single thickness of barrier layer material can be utilized, such thick porous materials are not readily available commercially. Therefore, in a preferred embodiment a multiplicity of barrier layers, 3, is utilized, the individual layers being adhesively bonded to one another.

In operation a vacuum is drawn on the sterilizer autoclave. In the process air entrapped within the barrier layer, 3, is withdrawn. When steam is allowed to enter the autoclave it moves freely to the indicator layer, 2, through the porous barrier layer, 3, from the bottom and edges and causes a colour change in the indicator layer, 2. If an adequate vacuum is not achieved the thermochromic ink of the indicator layer will not change colour uniformly or completely as a result of entrapped air which prevents steam from migrating freely through the porous barrier layer, 3. While leaving the device in the steam autoclave for an extended period will ultimately result in a colour change, the test requirements must be met during the normal sterilization cycle for the type of autoclave used.

To operate properly the barrier layer, 3, must have sufficient bulk to offer resistance to flow of steam in the absence of a vacuum. Conversely under vacuum it must be sufficiently porous to the steam to result in an appropriate colour change during the test cycle. This predetermined porosity requirement can be fulfilled in a number of ways.

Illustrative non-limiting examples of barrier layers useful in the practice of this invention are a 120 point solid bleached sulphate, coated two sides, sold by RUPACO Paper Corp. of Edison, New Jersey. This product has a basis weight of about 400 pounds per thousand square feet (1.95 kg.m$^{-2}$), and comprises 5 layers of 24 point paper, each having a basis weight of 80 pounds (0.39 kg.m$^{-2}$), laminated together. Another suitable a composition comprising three layers of Twice White II™, a 48 mil (1.2 mm) paper having a basis weight of 156.5 pounds per 1000 square feet (0.76 kg.m$^{-2}$) (basis weight of about 460 lbs (2.25 kg.m$^{-2}$) for three layer composite of 144 points) supplied by Salwen Paper Co., Inc. of Edison, New Jersey. Those skilled in the art will appreciate that for paper one point of thickness equals one mil (0.025 mm). The three sheets were bonded to one another using an acrylic emulsion adhesive spray. The porosity of both of these laminates give the desired barrier properties so that the indicator layer, 2, ink will change colour uniformly and completely if an adequate vacuum, e.g., 28 inches of mercury (95 kPa), is draw on the autoclave. In the absence of an adequate vacuum, the colour change will be incomplete.

Generally, where paper board is used an adequate range for thickness and basis weight is a thickness of about 110 to about 150 mils (2.7 to 3.8 mm), and a basis weight of about 390 to about 475 pounds per thousand square feet (1.9 to about 2.3 kg.m$^{-2}$); preferably about 120 to about 144 mils (about 3 to about 3.6 mm) in thickness and a basis weight of about 400 to about 460 pounds (1.9 to about 2.3 kg.m$^{-2}$). It will be appreciated by those skilled in the art having access to this disclosure that the higher basis weights are associated with the greater thickness of the barrier layer.

The products were tested by applying the indicator ink in a chevron pattern on the top surface of the laminate and adhesive bonding an overlayer of polyester (polyethylene glycol terephthalate) on the ink surface. The test specimen was tested with controlled leaks of 0.5 l./min., 1 l./min., 2 l./min., and no leak at a vacuum of 28 inches of mercury (95 kPa). The test time was 3.5 minutes at 270°F (132°C). The colour change is from purple to green. The first three samples showed an incomplete colour change, while the latter sample changed completely and uniformly from purple to green.

Using these materials with their predetermined porosity as a standard it is within the skill of those in the art to select, without undue experimentation, similar commercially available papers which will perform in the same manner as the standard. Additionally, other porous materials such as felt fabrics and porous open celled sponges of cellulose or polymeric open celled foam materials can be utilized in preparing a porous barrier layer, 3.

Where the barrier layer being tested exhibits too high a porosity, a correction can be made by adhering a rate controlling polymer film to the bottom of the barrier layer. Suitable rate controlling films include polypropylene and nylon films of about 0.5 to about 2 mils (0.01 to 0.05 mm), eg about 1 mil (0.025 mm).

Any adhesive which is water resistant and can withstand the sterilization process temperatures can be utilized. It is preferred that the adhesive be applied as a spray so that minimal amounts are used. In this way the adhesive will not contribute significantly to the barrier properties of the porous barrier. It will be appreciated by those skilled in the art having access to this disclosure that in making the porosity comparison with the standard the adhesive barrier properties will fortuitously be taken into account. Suitable adhesives include acrylic adhesives and silicone adhesives. A commercially available adhesive suitable for use in the practice of this invention is an acrylic adhesive spray sold by 3M under its trademark SUPER 77 Spray adhesive

Any thermochromic ink which will change colour under the sterilization process conditions can be used. It is essential for the practice of this invention that the colour change to be observed not occur in the absence of steam. A common prior art ink which changes colour under the sterilization condition in the presence of steam comprises a mixture of lead carbonate and sulphur. See for example US-2118144. While this ink can be used it is not preferred. The colour change is not sharp, and the difference between a good and a poor vacuum can be difficult to discern. The preferred ink utilizes chromium trichloride as the thermochromic compound. The colour change is from purple to green. Hence, it is readily discernable. The indicating composition will of course be formulated for ease of application to the indicator layer.

While the term thermochromic ink usually connotes an ink which will change colour in response to a particular temperature or range of temperatures, as used in the specification and claims, the term "thermochromic ink" means an ink composition which will exhibit colour change responsive to temperature and have at least one colour state which requires the presence of steam for its formation. Illustrative non-limiting examples of such thermochromic inks are compositions containing chromium trichloride and mixtures of lead carbonate and sulphur. Typically, the reactive thermochromic chemical component of the ink can comprise about 20 to about 40 weight percent of the thermochromic ink composition, e.g. 30 wt.%. It will be appreciated that the concentration is a matter of preference depending on the intensity of colour desired.

Ink formulations useful in the practice of this invention are well known in the art. They can contain vehicles, fillers, brighteners, opacifiers, binders and thickeners. See for example US-3684737, US-3360338 and US-3360337 all of which are incorporated herein by reference.

The ink formulations of this invention are preferably water insoluble compositions, and usually slurries of insoluble components together with the thermochromic material of this invention, solvents, plasticisers and binders. Illustrative non-limiting examples of solvents useful in the practice of this invention are propyleneglycol methyl ether, propyleneglycol ethyl ether, and Cellosolve™ solvent manufactured by GALLADE CHEMICAL CORP., a 2-ethoxyethanol. A typical solvent for the ink compositions is a propyleneglycol methyl ether sold under the trademark Propasol M manufactured by Unical Corporation. The solvent content of the ink will depend on the desired viscosity for a given method of application. Generally, it can comprise about 15.0 to about 45.0 weight percent of the composition, typically about 20 to about 40 wt.%, e.g., 35 wt. %.

Suitable binders for the thermochromic inks of this invention include water insoluble modified cellulose compositions. Illustrative, non-limiting examples of water insoluble binders useful in the practice of this invention are ethylhydroxyethyl cellulose (EHEC), ethylhydroxymethyl cellulose, Rohm & Haas Acryloid DM-55, an isobornyl methacrylate copolymer and mixtures thereof. The binder can comprise about 3.50 to about 6.0 weight percent of the thermochromic ink composition, typically about 4.0 to about 5.0 , e.g., about 4.5 weight percent.

The brightener will be, generally, an inert inorganic oxide which is white in colour and imparts to the ink a brighter colour than will be achieved without it. Illustrative non-limiting examples of brighteners useful in the practice of this invention are antimony trioxide and titanium dioxide. The brightener can comprise about 5 to about 20 wt.%, typically about 8 to about 18 wt.%, the thermochromic ink composition, e.g., 10 wt.%.

The opacifier will be, generally, a water insoluble, inorganic salt of a weak acid which is white in colour. Illustrative, non-limiting examples of such opacifiers are calcium carbonate, barium carbonate and lead carbonate. The opacifier can comprise about 12 to about 35 wt.%, typically about 15 to about 25 wt. % of the thermochromic ink composition, e.g., 18 wt.%.

Illustrative non-limiting examples of plasticisers are dialkyl phthalates, e.g., diethyl phthalate, dioctyl phthalate; bis(hydroxymethyl) oleyl oxazoline. The plasticizer can comprise about 2 to about 8 wt.% of the composition, typically about 2.5 to about 6.0 wt.%, e.g. about 5.0 wt.%.

The amount of brightener and opacifier used will effect the colour shade of the ink and may be varied to suit the colour preference of the user.

Where the ink is applied by silk screening it is preferable to include a fixative to reduce the drying rate of the ink. Illustrative, non-limiting examples of fixatives are glycerine, polyalkylene glycols and alkoxylated phenol, e.g., polyethylene glycol, polypropylene glycol and ethoxylated phenol. Generally, the fixative is an oxygen containing organic compound, e.g., hydroxyl or ether containing compounds, compatible with the solvents, and having a higher vapour pressure. The usually will not evaporate to dryness. These compound are utilized at about 2 to about 8.0 wt.%, typically about 2.5 to about 6 wt.%., e.g., 3.8 wt.%.

An illustrative thermochromic ink composition is shown in Table I,

TABLE I

| COMPONENT | GRAMS |
|---|---|
| 2-ethoxy ethanol | 32.92 |
| Alkaterge T (plasticiser)* | 2.19 |
| Chromium trichloride | 18.11 |
| Antimony trioxide | 8.78 |
| Calcium carbonate | 18.11 |
| V-125 (varnish) | 2.19 |
| Diethyl phthalate | 2.89 |
| V-126 (varnish) | 10.97 |
| Polyethylene glycol (mw 200) | 3.84 |

* - a plasticizer manufactured by Angus Chemical Company comprising bis(hydroxymethyl) oleyl oxazoline.

Two varnishes, V-125 and V-126 were prepared as blends for addition to the formulation. Varnish V-125 comprised 60 wt.% Acryloid DM 55 and 40 wt.% Propasol M. Varnish V-126 comprised 30 wt.% EHEC and 70 wt.% Propasol M.

The components were blended together and ball milled until the composition was uniform. Generally, ball milling takes about one hour. The ink was then applied to the indicator layer substrate by silk screening. However, any suitable method of application may be utilized, e.g., off set printing.

The ink can be applied to the indicator layer substrate in any pattern desired. As was pointed out above, the device need not have a separate indicator layer. The thermochromic ink can be imprinted in a suitable pattern on the upper surface of the barrier layer. Figure 2 is illustrative of a suitable prior art chevron pattern.

**Claims**

1. An improved sterilization autoclave vacuum testing device comprising a transparent gas impervious layer adhered to an indicator layer comprising a steam porous medium having imprinted there on a pattern utilizing a thermochromic ink composition, the indicator layer being adhesively bonded to a porous barrier layer substrate having a predetermined porosity, sufficient to prevent a complete and uniform colour change of the thermochromic ink at a vacuum of at least about 711 mm of mercury (95 kPa) at a predetermined temperature.

2. A device as claimed in claim 1, in which the gas impervious layer comprises polyester film.

3. A device as claimed in claim 1 or claim 2, in which the gas impervious layer is adhered to the indicator layer by adhesive bonding.

4. A device as claimed in any one of claims 1 to 3, in which the porous barrier layer comprises a multiplicity of layers of paper adhesively bonded to one another.

5. A device as claimed in claim 3 or claim 4, in which the adhesive is an acrylic adhesive.

6. An improved sterilization autoclave vacuum testing device comprising a composite structure of:
   (a) transparent gas impervious layer comprising a polyester film having a thickness of about 0.01 mm to about 0.03 mm;

6

(b) an indicator layer comprising a steam porous medium having imprinted there on a pattern utilizing a thermochromic ink composition, the gas impervious layer being adhesively bonded to the indicator layer, preferably by means of an acrylic adhesive; and

(c) a porous barrier layer having a predetermined porosity, barrier layer being adhesively bonded to the indicator layer, preferably by means of an acrylic adhesive.

7. A device as claimed in claim 6, in which the porous barrier layer comprises at least two layers of a paper board.

8. A device as claimed in claim 7, in which each of the layers of paper board has a thickness of at least about 1 mm (for example about 1.2 mm), and a basis weight of at least about $0.5$ kg.m$^{-2}$ (for example about $0.76$ kg.m$^{-2}$), the layers being bonded together by means of an adhesive.

9. An improved sterilization autoclave vacuum testing device comprising a transparent gas impervious layer having imprinted on its underside a pattern utilizing a thermochromic ink composition, the gas impervious layer being adhesively bonded to a porous substrate having a predetermined porosity.

10. An improved sterilization autoclave vacuum testing device comprising a transparent gas impervious layer and a porous barrier layer having a predetermined porosity, the barrier layer having imprinted on an upper surface a pattern utilizing a thermochromic ink composition, the gas impervious layer being bonded to the porous barrier layer substrate and overlaying the thermochromic ink pattern.

11. A device as claimed in claim 9 or claim 10, in which the porous barrier layer comprises at least three layers of a paper board.

12. A device as claimed in claim 11, in which each of the layers has a thickness of at least about 1 mm (for example about 1.2 mm), and a basis weight of at least about $0.5$ kg.m$^{-2}$ (for example about $0.76$ kg.m$^{-2}$), the layers being bonded together by means of an adhesive.

13. A device as claimed in claim 12, in which the thickness of the laminate is from about 2.7 to about 3.8 mm, preferably from about 3 to about 3.6 mm, and the basis weight of the laminate is from about 1.9 to about 2.3 kg.m$^{-2}$, preferably from about 1.95 to about 2.2 kg.m$^{-2}$.

14. A device as claimed in any preceding claim, in which the thermochromic ink comprises chromium trichloride.

15. A device as claimed in any preceding claim, in which the thermochromic ink composition comprises at least one of a thermochromic compound, a brightener, an opacifier, a binder and a vehicle for the ink composition.

16. A device as claimed in claim 15, in which the brightener comprises antimony trioxide, the opacifier comprises calcium carbonate, the binder comprises ethylhydroxyethyl cellulose and the vehicle comprises propyleneglycol methyl ether.

17. A device as claimed in any preceding claim, in which the ink pattern comprises a chevron pattern.

# FIG.1

# FIG.2

ATI  Bowie-Dick Test Card

This Side up

**European Patent Office**

# EUROPEAN SEARCH REPORT

Application Number

EP 94 30 4125

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | GB-A-1 215 891 (ROY HENRY MAXWELL)<br>* claims * | 1-17 | A61L2/26 |
| D,Y | GB-A-2 180 933 (WARNER LAMBERT COMPANY.)<br>* claims; figures *<br>& US-A-4 692 307 | 1-17 | |
| A | EP-A-0 428 245 (PYMAH CORPORATION)<br>* claims; figures * | 1 | |
| A | EP-A-0 202 724 (WARNER-LAMBERT COMPANY)<br>* claims; figures * | 1 | |
| A | WO-A-87 04931 (THE VICTORIA UNIVERSITY OF MANCHESTER.)<br>* claims * | 1 | |
| D,A | US-A-4 576 795 (LORAN H. BRUSO.)<br>* figures 1-7 * | 1 | |
| | | | **TECHNICAL FIELDS SEARCHED (Int.Cl.5)**<br><br>A61L |

The present search report has been drawn up for all claims

| Place of search | Date of completion of the search | Examiner |
|---|---|---|
| THE HAGUE | 22 September 1994 | ESPINOSA, M |